# EUROPEAN PATENT SPECIFICATION

(11) **EP 2 983 493 B1**
(45) Date of publication and mention of the grant of the patent: **05.06.2019**
(21) Application number: 14769756.9
(22) Date of filing: 11.02.2014
(51) Int. Cl.: A23K 10/00, G01N 33/02, G01N 1/40, G01N 21/3563, G01N 21/359, C12Q 1/37

(54) **METHOD FOR ANALYZING ANIMAL FEED**
VERFAHREN ZUR ANALYSE VON TIERFUTTER
PROCÉDÉ POUR ANALYSER UNE NOURRITURE POUR ANIMAL

(30) Priority: 15.03.2013 US 201361787842 P; 17.12.2013 US 201314109359
(43) Date of publication of application: 17.02.2016
(73) Proprietor: Alltech, Inc., Nicholasville, KY 40356 (US)
(72) Inventor: MCKINNEY, Kyle, Lexington, KY 40514 (US); LOVELL, Allyson, Lexington, KY 40502 (US); HENRY, Benjamin, Shelbyville, KY 40065 (US); BECKER, Patrick, Lexington, KY 40514 (US); TIMMONS, Rebecca, A., Lexington, KY 40503 (US)
(74) Representative: Grünecker Patent- und Rechtsanwälte PartG mbB
(86) International application number: PCT/US2014/015729
(87) International publication number: WO 2014/149239

(56) References cited:
- US-A- 6 166 382
- US-A1- 2009 092 715
- US-B1- 6 532 420
- US-B1- 7 490 437
- BOISEN ET AL: "In vitro analyses for predicting standardised ileal digestibility of protein and amino acids in actual batches of feedstuffs and diets for pigs", LIVESTOCK SCIENCE, ELSEVIER, AMSTERDAM, NL, vol. 109, no. 1-3, 29 April 2007 (2007-04-29), pages 182-185, XP022053301, ISSN: 1871-1413, DOI: 10.1016/J.LIVSCI.2007.01.141
- Balthrop Et Al: "QUALITY ASSURANCE FOR ANIMAL FEED ANALYSIS LABORATORIES" In: "FAO ANIMAL PRODUCTION AND HEALTH MANUAL", 2011, XP055279521, pages 1-178, * page 23, last paragraph - page 24, paragraph 7 * * page 167, last paragraph - page 174, paragraph 4 *
- ERNESTO A RESTAINO ET AL: "Prediction of the Nutritive Value of Pasture Silage by Near Infrared Spectroscopy (NIRS)", CHILEAN JOURNAL OF AGRICULTURAL RESEARCH, vol. 69, no. 4, 2009, XP055300248, DOI: 10.4067/S0718-58392009000400011
- BALTHROP ET AL.: 'Quality Assurance for Animal Feed Analysis Laboratories (Manual' FAO ANIMAL PRODUCTION AND HEALTH MANUAL 2011, ROME, pages 1 - 178, XP055279521
- MENTINK ET AL.: 'Utility of Near-Infrared Reflectance Spectroscopy to Predict Nutrient Composition and In Vitro Digestibility of Total Mixed Rations.' J. DAIRY SCI. vol. 89, no. 6, June 2006, pages 2320 - 2326, XP026957062

## Description

### FIELD OF THE INVENTION

The present invention relates to a method for analyzing animal feeds. In particular, there are described in vitro systems and methods for analyzing animal feed for metabolism of nutrients and energy sources.

### CROSS REFERENCE TO RELATED APPLICATIONS

This application is being filed on 11 February 2014, as a PCT International Patent application and claims priority to U.S. Patent Application Serial No. 61/787,842 filed on 15 March 2013, and U.S. Patent Application Serial No. 14/109,359 filed on 17 December 2013.

### BACKGROUND OF THE INVENTION

Most animal feeds have as a primary goal the provision of at least a minimum requirement of nutrition to sustain the animals to which it is fed.

Livestock (e.g., bovines, porcines, poultry, fish, etc.) have been selected over the past 20-50 years for specific characteristics such as growth, leanness, and metabolism efficiency. Thus, over the last fifty years, approaches toward providing animal nutrition have changed. No longer are animals fed whatever forage or other material that may be available. Instead, the diets of animals are closely monitored for total nutrition value and cost. Very often, animals on specific diets are monitored for quality and performance characteristics with the nutritional components of the feed being adjusted to maximize nutrition value of the feed and optimization of animal performance characteristics.

A need exists to analyze feed for levels of nutrients and to determine if the feed meets energy and nutrient requirements of the animal it is intended for. Preferred analysis methods are efficient, accurate, and cost effective.

### SUMMARY OF THE INVENTION

The present invention relates to a method for analyzing animal feeds according to claim 1. In particular, there are described in vitro systems and methods for analyzing animal feed for metabolism of nutrients and energy sources.

For example, in some embodiments, the present invention provides a method of analyzing animal feed, comprising: a) performing in vitro digestion on a sample of the animal feed to generate digested animal feed; b) analyzing the digested animal feed using spectroscopy (e.g., near infrared spectroscopy) to generate spectral data; and c) identifying peaks in the spectral data to determine peak information for the animal feed. In some embodiments, the peak information comprises identity and quantity of residual components of the animal feed following in vitro digestion. In some embodiments, the residual components are one or more of phosphorous, protein, carbohydrates, or gross energy. In some embodiments, the animal feed comprises an enzyme (e.g., a digestive enzyme or an enzyme involved in digesting animal feed). Exemplary enzymes include, but are not limited to, proteases, fungal proteases, cellulases, xylanases, phytase, acid phosphatases, beta-glucanase, pectinase, or alpha amylase. In some embodiments, the identifying is used to determine the effect of the enzyme on the digestibility and/or bioavailability of the animal feed. In some embodiments, the identifying comprises the use of a computer and computer software, wherein the software utilizes previously generated peak information to determine the identity and quantification of a particular peak.

A system for performing the method can comprise :a) an in vitro digestion apparatus for performing in vitro digestion on a sample of an animal feed to generate digested animal feed; b) a spectrometer for generating spectra of the digested animal feed; and c) a computer and computer software for identifying and quantifying peaks on the spectra. The computer software determines the effect of said enzyme on the digestibility of the animal feed.

Additional embodiments are described herein.

### DESCRIPTION OF THE DRAWINGS

Figure 1 shows a schematic of methods of embodiments of the present disclosure.
Figure 2 shows an exemplary NIR spectrum.
Figure 3 shows the accuracy of an exemplary model of embodiments of the present disclosure.

### DEFINITIONS

As used herein, the term w/w (weight/weight) refers to the amount of a given substance in a composition on weight basis. For example, an animal feed comprising 0.02% w/w dietary feed supplement means that the mass of the dietary feed supplement is 0.02% of the total mass of the animal feed (e.g., 200 grams of dietary feed supplement composition of the invention in 907,200 grams of animal feed).

As used herein, the term "algal" or "algae" refers to any single- or multicellular organism capable of being cultivated in media comprising dilute, full-strength, or concentrated sea water or naturally found in marine waters and that can be propagated through culturing or fermentation methods.

As used herein, the term "algal meal" refers to a preparation of algal material.

As used herein, the term "preservative" refers to an agent that extends the storage life of food and non-food products by retarding or preventing deterioration of flavor, odor, color, texture, appearance, nutritive value, or safety. A preservative need not provide a lethal, irreversible action resulting in partial or complete microbial cell destruction or incapacitation. Sterilants, sanitizers, disinfectants, sporicides, virucides and tuberculocidal agents provide such an irreversible mode of action, sometimes referred to as "bactericidal" action. In contrast, a preservative can provide an inhibitory or bacteriostatic action that is reversible, in that the target microbes can resume multiplication if the preservative is removed. The principal differences between a preservative and a sanitizer primarily involve mode of action (a preservative prevents growth rather than killing microorganisms) and exposure time (a preservative has days to months to act whereas a sanitizer has at most a few minutes to act).

As used herein, the term "*Aspergillus niger* extract" or "fungal extract" or "*Aspergillus niger* fermentation extract" or "fungal fermentation extract" refers to a product of fungal fermentation. In some embodiments, the organism used for fungal fermentation is in the genera *Aspergillus.* In some embodiments, the product of fungal fermentation comprises at least one enzyme. In some embodiments, the enzymatic activity comprises protease activity. Other enzymatic or non-enzymatic activities, properties, or components may be present in the product of fungal fermentation. Such activities, properties, or components include but are not limited to cellulose-degrading activity, secondary metabolites, antibiotic activity, growth-promoting activity, or facilitation of digestion particularly within the gastrointestinal system of a livestock species.

As used herein, the term "purified" or "to purify" refers to the removal of components from a sample. For example, yeast cell walls or yeast cell wall extracts are purified by removal of non-yeast cell wall components (e.g., plasma membrane and/or yeast intracellular components); they are also purified by the removal of contaminants or other agents other than yeast cell wall. The removal of non-yeast cell wall components and/or non-yeast cell wall contaminants results in an increase in the percent of yeast cell wall or components thereof in a sample.

As used herein, the term "in vivo" refers to studies and/or experiments conducted within a living organism, occurring within a biological organism.

As used herein, the term "in vitro" refers to an artificial environment outside the living organism and to biological processes or reactions that would normally occur within an organism but are made to occur in an artificial environment. In vitro environments can comprise, but are not limited to, test tubes and cell culture.

As used herein, the term "analyte" refers to an atom, a molecule, a grouping of atoms and/or molecules, a substance, or chemical constituent. An analyte, in and of itself cannot be measured; rather, aspects or properties (physical, chemical, biological, etc.) of the analyte can be determined using an analytical procedure, such as HPLC. For example, one cannot measure a "chair" (analyte-component) in and of itself, but, the height, width, etc. of a chair can be measured.

As used herein, the term "bioavailability" refers to the fraction of a molecule or component that is available to an organism or reaches the systemic circulation. When a molecule or component is administered intravenously, its bioavailability is 100%. However, when a molecule or component is administered via other routes (such as orally), its bioavailability decreases (due to incomplete absorption and first-pass metabolism). In a nutritional setting, bioavailability refers to the rates of absorption and utilization of a nutrient. Different forms of the same nutrient, for example, may have different bioavailabilities.

As used herein, the term "absorb" refers to the process by which a material "takes in" or "sucks up" another substance. For example, "absorption" may refer to the process of absorbing or assimilating substances into cells or across the tissues and organs through diffusion or osmosis (e.g. absorption of nutrients by the digestive system or absorption of drugs into the blood stream).

As used herein, the term "adsorption" refers to a process that occurs when a material is sequestered by, and/or accumulates on the surface of, a solid or a liquid (sequestrant and/or adsorbent) (e.g. thereby forming a film of molecules or atoms (the adsorbate)).

As used herein, the term "digest" refers to the conversion of food, feedstuffs, or other organic compounds into absorbable form; to soften, decompose, or break down by heat and moisture or chemical action.

As used herein, "digestive system" refers to a system (including gastrointestinal system) in which digestion can or does occur.

As used herein, the term "feedstuffs" refers to material(s) that are consumed by animals and contribute energy and/or nutrients to an animal's diet. Examples of feedstuffs include, but are not limited to, Total Mixed Ration (TMR), forage(s), pellet(s), concentrate(s), premix(es) coproduct(s), grain(s), distiller grain(s), molasses, fiber(s), fodder(s), grass(es), hay, kernel(s), leaves, meal, soluble(s), and supplement(s).

As used herein, the terms "food supplement" "dietary supplement" "dietary supplement composition" and the like refer to a food product formulated as a dietary or nutritional supplement to be used as part of a diet, e.g. as an addition to animal feed. Exemplary dietary supplement compositions are described herein.

As used herein, the term "omega-3 fatty acid" refers to polyunsaturated fatty acids that have the final double bond in the hydrocarbon chain between the third and fourth carbon atoms from the methyl end of the molecule. Non-limiting examples of omega-3 fatty acids include, 5,8,11,14,17-eicosapentaenoic acid (EPA), 4,7,10,13,16,19-docosahexanoic acid (DHA) and 7,10,13,16,19-docosapentanoic acid (DPA).

As used herein, the term "animal" refers to those of kingdom Animalia. This includes, but is not limited to livestock, farm animals, domestic animals, pet animals, marine and freshwater animals, and wild animals.

As used herein, the term "toxic" refers to any detrimental, deleterious, harmful, or otherwise negative effect(s) on a subject, a cell, or a tissue as compared to the same cell or tissue prior to the contact or administration of the toxin/ toxicant.

As used herein, the term "acid" as used herein refers to any chemical compound that can donate proton(s) and/or accept electron(s). Acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, sulfonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

As used herein, the term "base" refers to any chemical compound that can accept proton(s) and/or donate electron(s) or hydroxide ions. Bases include, but are not limited to, alkali metal (e.g., sodium) hydroxides, alkaline earth metal (e.g., magnesium) hydroxides, ammonia, and compounds of formula NW₄⁺, wherein W is C₁₋₄ alkyl, and the like.

As used herein, the term "salt" refers to compounds that may be derived from inorganic or organic acids and bases. Examples of salts include, but are not limited to, acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, chloride, bromide, iodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like.

As used herein, the term "antifoaming agent" refers to an additive used to prevent formation of foam or is added to break foam already formed. An "antifoaming agent" also referred to as "antifoamer" or "defoamer" refers to an additive which reduces the surface tension of a solution or media or emulsion or broth in fermenters because of aeration or agitation, thus inhibiting or modifying the formation of a foam. Commonly used agents are insoluble oils, dimethyl polysiloxanes and other silicones, certain alcohols such as stearyldecanol, octal decanol, sulphonates, stearates and glycols.

As used herein, the term "cell" refers to an autonomous self-replicating unit that may exist as functional independent unit of life (as in the case of unicellular organism, e.g. yeast), or as sub-unit in a multicellular organism (such as in plants and animals) that is specialized into carrying out particular functions towards the cause of the organism as a whole. There are two distinct types of cells: prokaryotic cells and eukaryotic cells.

As used herein, the term "eukaryote" refers to organisms whose cells are organized into complex structures enclosed within membranes. "Eukaryotes" are distinguishable from "prokaryotes." The term "prokaryote" refers to organisms that lack a cell nucleus or other membrane-bound organelles. The term "eukaryote" refers to all organisms with cells that exhibit the typical characteristics of eukaryotes, such as the presence of a true nucleus bounded by a nuclear membrane, within which lie the chromosomes, the presence of membrane-bound organelles, and other characteristics commonly observed in eukaryotic organisms. Thus, the term includes, but is not limited to such organisms as fungi, protozoa, and animals.

As used herein, the term "concentration" refers to the amount of a substance per defined space. Concentration usually is expressed in terms of mass per unit of volume. To dilute a solution, one must add more solvent, or reduce the amount of solute (e.g., by selective evaporation, spray drying, freeze drying, e.g., concentrated yeast cell wall extract or concentrated modified yeast cell wall extract). By contrast, to concentrate a solution, one must add more solute, or reduce the amount of solvent.

As used herein, the term "layer" refers to a usually horizontal deposit organized in stratum of a material forming an overlying part or segment obtained after separation by centrifugation in relation with the density properties of the material.

As used herein, the term "harvest" refers to the act of collecting or bringing together materials that have been produced (e.g. bringing together materials produced during yeast production).

As used herein, the term "drying" refers to spray drying, freeze drying, air drying, vacuum drying or any other kind of process that reduces or eliminates liquid in a substance.

As used herein, the term "spray drying" refers to a commonly used method of drying a substance containing liquid using hot gas to evaporate the liquid to reduce or eliminate liquid in the substance. In other words the material is dried by way of spraying or atomizing into a draft of heated dry air.

As used herein, the term "freeze-drying" and the term "lyophilization" and the term "cryodesiccation" refer to the removal of a solvent from matter in a frozen state by sublimation. This is accomplished by freezing the material to be dried below its eutectic point and then providing the latent heat of sublimation. Precise control of heat input permits drying from the frozen state without product melt-back. In practical application, the process is accelerated and precisely controlled under reduced pressure conditions.

As used herein, the term "dry free flowing powder" refers to a free flowing dry powder, e.g. a powder that can be poured from a container, bag, vessel etc without hindrance of large clumps.

As used herein, the term "grinding" refers to reducing particle size by impact, shearing, or attrition.

As used herein, the term "washing" refers to the removal or cleansing (e.g., using any type of solute (e.g. distilled water, buffer, or solvent) or mixture) of impurities or soluble unwanted component of a preparation (e.g., a yeast cell wall extract may be washed to remove non-yeast cell wall components from the sample).

As used herein, the term "enzyme" refers to as a protein or protein-based molecule with a characteristic sequence of amino acids that fold to produce a specific three-dimensional structure which gives the molecule unique properties and that acts as a catalyst or a chemical for specific chemical reactions, converting a specific set of reactants (called substrates) into specific products.

As used herein, the term "peptide," the term "polypeptide" and the term "protein" refer to a primary sequence of amino acids that are joined by covalent "peptide linkages." Generally, a peptide consists of a few amino acids, typically from 2-50 amino acids, and is shorter than a protein. The term "polypeptide" encompasses peptides and proteins. Peptides, polypeptides or proteins can be synthetic, recombinants or naturally occurring. A synthetic peptide is produced by artificial means in vitro (e.g., is not produced in vivo).

As used herein, the term "proteases" refers to any of various enzymes, including the endopeptidases and exopeptidases, that catalyze the hydrolytic breakdown of proteins into peptides or amino acids.

As used herein, the term "lysis" refers to the disintegration or rupture of the yeast cell membrane and yeast cell wall resulting in the release of the intracellular components. As used herein, "lysis" occurs as a result of physical, mechanical, enzymatic (including autolysis and hydrolysis) or osmotic mechanisms (including "alcohol shocking" and hydrolysis).

As used herein, the term "autolysis" refers to the breakdown of a part or whole cell or tissue by self-produced agents such as, e.g., enzymes.

As used herein, the term "hydrolysis", refers to the process of splitting a compound into fragments with the addition of water (e.g., that is used to break down polymers into simpler units (e.g. starch into glucose)).

As used herein, the term "sample" is used in a broad sense including a specimen or culture obtained from any source, as well as biological and environmental samples. Biological samples may be obtained from animals (including humans) and encompass fluids, solids, tissues, and gases. Biological samples include blood products, such as plasma, serum and the like. Environmental samples include environmental material such as surface matter, soil, water, crystals and industrial samples.

As used herein, the term "complex" refers to an entity formed by association between two or more separate entities (e.g., association between two or more entities wherein the entities are the same or different (e.g., same or different chemical species). The association may be via a covalent bond or a non-covalent bond (e.g., via van der Waals, electrostatic, charge interaction, hydrophobic interaction, dipole interaction, and/or hydrogen bonding forces (e.g., urethane linkages, amide linkages, ester linkages, and combination thereof)).

As used herein, the term "antioxidant" refers to a molecule capable of slowing or preventing the oxidation of other molecules.

As used herein, the term "vitamin E" also referred to as "VE" refers to the collective name for a set of 8 related α-, β-, γ-, and δ-tocopherols and the corresponding four tocotrienols, which are fat-soluble vitamins with antioxidant properties.

As used herein, the term "vitamin C" refers to an essential nutrient for humans, a large number of higher primate species, a small number of other mammalian species (notably guinea pigs and bats), a few species of birds, and some fish.

As used herein, the term "ascorbate" refers to (an ion of ascorbic acid) is required for a range of essential metabolic reactions in all animals and plants.

As used herein the term, "preservative agent" and then term "preservative" refer to keeping intact, free from decay and maintaining or saving from decomposition.

As used herein the term, "livestock" also referred to as "livestock species" and also referred to "domestic livestock" and also referred to as "commercially raised animals" refers to a domesticated animal intentionally reared in an agricultural or aquaculture setting to produce things such as food or fiber, or for its labor.

As used herein the term, "TAC" refers to total antioxidant capacity. TAC can refer to the spectrum of antioxidant activity against various reactive oxygen/nitrogen radicals. A number of different types of assays can be utilized to determine TAC including Brunswick total antioxidant capacity assays (e.g., focused on the non-enzymatic antioxidants against peroxyl radical (ROO), hydroxyl radical (HO), singlet oxygen (102) and peroxynitrite (ONOO-).

### DETAILED DESCRIPTION OF THE INVENTION

The present invention relates to a method for analyzing animal feeds. In particular, there are described in vitro systems and methods for analyzing animal feed for metabolism of nutrients and energy sources.

Current methods of analyzing animal feed for nutrients and energy sources involve procedures that require up to two weeks of preparatory work and use several separate pieces of equipment. Some methods involve in vivo digestion characteristics analyzed using near infrared spectroscopy. The models developed using such methods only give a predictor of an initial feed, which does not include the effects certain enzymes may have on improving digestion. These solutions do not use an animal in vitro procedure nor do they continuously mimic digestion. Most techniques are only applicable to one specific feed. There is not a procedure at the current time that can give results for a multitude of feeds and enzymes in an efficient manner.

Accordingly, in some embodiments, the present invention provides an efficient way to analyze feed (e.g., animal feed) for enzymatic effects on gross energy, digestible energy, phosphorous release, sugar release, and to determine if an additive enzyme is present in the feed. This systems and methods described herein can analyze a multitude of feeds for multiple components and can be updated rapidly without undergoing in vivo trials.

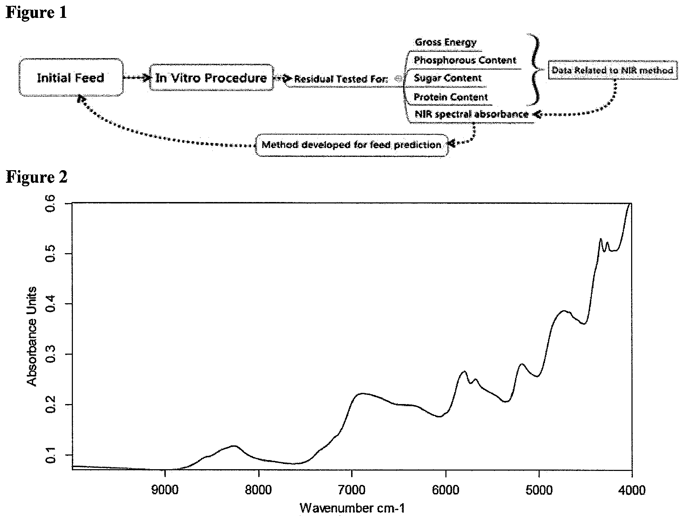

Embodiments of the present invention are described in Figure 1 and Example 1 below. Example 1 describes the development of a database and/or model that identifies and characterizes (e.g., quantifies) spectral data. For example, in some embodiments, methods utilize in vitro digestion, followed by analysis with a near infrared spectroscopy device and analytic analysis. In some embodiments, analytical analysis identifies residual protein, phosphorous, carbohydrates, and gross energy remaining after in vitro digestion. In some embodiments, a bomb calorimeter is used to assay for gross energy, a nitrogen combustion analyzer for protein, and minerals detector (e.g., inductively coupled plasma (ICP)) for phosphorous content. NIR is used to scan the digested material. The analytical results from a plurality of samples (e.g., 5 or more 10 or more, 20 or more, 50 or more, etc.) is used to correlated the NIR peaks with a particular component of the digested feed. A model that allows for identification and analysis of NIR spectra is then generated from the correlation. This model or database is then utilized to identify and quantify peaks without the need to conduct a full analytical analysis on each sample.

### I. Feed analysis

Thus, in some embodiments, the systems and methods of the present disclosure perform the following steps: a) feed is digested using an in vitro model; b) the dry matter collected is analyzed using spectroscopy (e.g., NIR); and c) the described database/model is used to identify and quantify spectral peaks. The systems and methods described herein allow for rapid and accurate analysis of animal feed without extensive analytical analysis. In some embodiments, the results are used to predict the impact of a particular enzyme (e.g., digestive enzyme) on digestibility of a given feed formulation.

In vitro digestion is utilized to mimic animal digestion as closely as possible to what naturally occurs inside an animal, while providing consistent results under laboratory controlled conditions. The digestion procedure is important for the effects of the analyzed component (e.g., enzyme) to be fully realized.

In some embodiments, in vitro digestion includes a gastric digestion step, and a subsequent intestinal digestion step. In some embodiments, pepsin is included in the gastric digestion step and pancreatin is included in the intestinal digestion step. The present invention is not limited to a particular time course, pH, or digestion enzyme. The protocol can be altered based on the feed and desired levels of digestion. Exemplary in vitro digestion methods are described in U.S. Patents 6,750,035, 8,357,408 and 8,067,238 and Boisen, S. (1990). A Model for Feed Evaluation Based on In vitro Digestible Dry Matter and Protein. In: In vitro Digestion for Pigs and Poultry (M.F. Fuller, editor). Oxford University Press, Oxford, pp. 136-139.

In some embodiments, near infrared spectroscopy is used to analyze digested feed. The near infrared spectroscopy device scans the digested feed for components located within the digested feed and provides a quantitative number. This allows for an accurate projection of an enzyme's effects within an animal.

Thus, in some embodiments, the systems and methods described herein find use in determining if a particular feed additive aids or inhibits digestion of the feed in an animal. This information is useful in determining feed formulations in order to maximize bioavailability of feed.

The systems and methods described herein can be used for more than one specific feed and can also observe the effects of multiple enzymes on a digestive level. These results are obtained in a quick manner that provides information to users (e.g., farmers) relatable to a real world environment.

The systems and methods described herein are suitable for analyzing feed for a variety of enzymes. The components within a complex feed can be altered and can come from several sources.

### II. Feed

The present disclosure finds use in the analysis of any number of animal feeds and is not limited to analysis of a particular feed. Animal feed is any foodstuff that is used specifically to feed domesticated livestock (e.g., cattle, goats, sheep, horses, poultry, buffalo, alpaca, llamas, donkeys, mules, rabbits, and pigs). Animal feeds often include hay, straw, silage, compressed and pelleted feeds, oils and mixed rations, enzymes, and also sprouted grains and legumes. The worldwide animal feed industry consumed 635 million tons of feed in 2006, with an annual growth rate of about 2%. The use of agricultural land to grow feed rather than human food can be controversial; some types of feed, such as corn (maize), can also serve as human food, while others such as grass cannot.

In addition to providing an energy source to animals, animal feeds also provide nutrients utilized by the body to protect it from oxidative stress. For example, animal feeds often include antioxidants that are important for optimizing immunity, health and production. Animals that possess strong antioxidant potential (e.g., via consuming a diet rich in antioxidants) results in animals that are better equipped to handle stress and perform to their full potential.

In some embodiments, feed includes selenium. The present invention is not limited by the type or source of selenium component. Indeed, a variety of different types and sources of selenium find use in the invention including but not limited to organic sources of selenium (e.g., selenized yeast, selenomethionine, etc.) as well as inorganic sources of selenium (e.g., selenium salt (e.g., sodium selenite, sodium selenate, cobalt selenite and cobalt selenate, selenic acid, selenious acid, selenium bromide, selenium chloride, selenium hexafluoride, selenium oxide, selenium oxybromide, selenium oxychloride, selenium oxyfluoride, selenium sulfides, selenium tetrabromide, selenium tetrachloride, selenium tetrafluoride, etc.). In a preferred embodiment, the selenium component is SEL-PLEX (Alltech, Nicholasville, KY).

In some embodiments, animal feeds include omega-3 fatty acids. The present invention is not limited to any particular omega-3 fatty acid. Indeed, a variety of omega-3 fatty acids find use in a dietary supplement composition of the invention including but not limited to α-Linolenic acid (ALA), Stearidonic acid (STD), Eicosatrienoic acid (ETE), Eicosatetraenoic acid (ETA), Eicosapentaenoic acid (EPA), Docosapentaenoic acid (DPA), Docosahexaenoic acid (DHA), Tetracosapentaenoic acid, and Tetracosahexaenoic acid (Nisinic acid). In a preferred embodiment, the omega-3 fatty acid is DHA. Similarly, the present invention is not limited to any particular source of omega-3 fatty acid. Indeed, a variety of sources of omega-3 fatty acids may be utilized including, but not limited to, algae (e.g., from algae meal), krill oil, or other source known to possess omega-3 fatty acids. In some embodiments, the omega-3 fatty acid is naturally produced (e.g., in a plant or animal cell). In some embodiments, the omega-3 fatty acid is synthetically generated.

In some embodiments, animal feeds comprise algal meal. In some embodiments, the algal meal is generated using organisms that produce high levels of fatty acids. In some embodiments, the algal meal is generated using a species with high yield of docosahexaenoic acid (DHA). In some embodiments, the algal species is within the Labyyrinthulomycetes (thraustochytrids, labyrinthulids). In some embodiments, the algal species is selected from, for example, one or more of the following: *Thraustochytrium* sp., *Thraustochytrium striatum, Thraustochytrium roseum*, *Thraustochytrium aureum*, *Schizochytrium limacinum*, *Crypthecodinium cohnii*, and *Aurantiochytrium* sp.

In some embodiments, animal feeds include antioxidants. In some embodiments, antioxidants are ascorbic acid. The present invention is not limited to any particular source or type of ascorbic acid (e.g., a sugar acid with antioxidant properties). In some embodiments, the ascorbic acid is vitamin C. In some embodiments, an ascorbate (e.g., ascorbic acid, mineral ascorbate salts, rose hips, acerola, and the like) is utilized.

In the case of an animal feed fed to animals, any animal feed blend known in the art can be used in accordance with the present invention such as rapeseed meal, cottonseed meal, soybean meal, and cornmeal, but soybean meal and cornmeal are particularly preferred. The animal feed blend is supplemented with a dietary supplement composition of the invention, but other ingredients can optionally be added to the animal feed blend. Optional ingredients of the animal feed blend include sugars and complex carbohydrates such as both water-soluble and water-insoluble monosaccharides, disaccharides and polysaccharides. Optional amino acid ingredients that can be added to the feed blend are arginine, histidine, isoleucine, leucine, lysine, methionine, phenylalanine, threonine, tryptophan, valine, tyrosine ethyl HCl, alanine, aspartic acid, sodium glutamate, glycine, proline, serine, cysteine ethyl HCl, and analogs, and salts thereof. Vitamins that can be optionally added are thiamine HCl, riboflavin, pyridoxine HCl, niacin, niacinamide, inositol, choline chloride, calcium pantothenate, biotin, folic acid, and vitamins A, B, K, D, E, and the like. Minerals, protein ingredients, including protein obtained from meat meal or fish meal, liquid or powdered egg, fish solubles, whey protein concentrate, oils (e.g., soybean oil), cornstarch, calcium, inorganic phosphate, copper sulfate, salt, and limestone can also be added. Any medicament ingredients known in the art can be added to the animal feed blend such as antibiotics.

In some embodiments, an animal feed comprises one or more of the following: Alfalfa (lucerne), Barley, Birdsfoot trefoil, Brassicas (e.g., Chau moellier, Kale, Rapeseed (Canola), Rutabaga (swede), Turnip), Clover (e.g., Alsike clover, Red clover, Subterranean clover, White clover), Grass (e.g., False oat grass, Fescue, Bermuda grass, Brome, Heath grass. Meadow grasses (from naturally mixed grassland swards), Orchard grass, Ryegrass, Timothy-grass), Corn (maize), Millet, Oats, Sorghum, Soybeans, Trees (pollard tree shoots for "tree-hay"), and Wheat.

Compositions of the invention may comprise one or more inert ingredients (e.g., if it is desirable to limit the number of calories added to the diet by the dietary supplement) when fed to the animals. For example, dietary supplement compositions and/or animal feeds or foodstuffs to which the dietary supplement composition of the invention is added may also contain optional ingredients including, for example, herbs, vitamins, minerals, enhancers, colorants, sweeteners, flavorants, inert ingredients, dehydroepiandosterone (DHEA), Fo-Ti or Ho Shu Wu (herb common to traditional Asian treatments), Cat's Claw (ancient herbal ingredient), green tea (polyphenols), inositol, kelp, dulse, bioflavinoids, maltodextrin, nettles, niacin, niacinamide, rosemary, selenium, silica (silicon dioxide, silica gel, horsetail, shavegrass, and the like), spirulina, zinc, and the like. Such optional ingredients may be either naturally occurring or concentrated forms.

In some embodiments, a dietary supplement composition of the invention is mixed with and/or combined with other foodstuffs (e.g., to generate an animal feed) including but not limited to, calcium phosphate or acetate, tribasic; potassium phosphate, dibasic; magnesium sulfate or oxide; salt (sodium chloride); potassium chloride or acetate; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D3; cyanocobalamin; sodium selenite; copper sulfate; vitamin A; inositol; potassium iodide. Suitable dosages for vitamins and minerals may be obtained, for example, by consulting the U.S. RDA guidelines.

In further embodiments, a dietary supplement composition of the invention or other foodstuff to which a dietary supplement composition is added to and/or combined with (e.g., to generate an animal feed) may include one or more food flavorings such as acetaldehyde (ethanal), acetoin (acetyl methylcarbinol), anethole (parapropenyl anisole), benzaldehyde (benzoic aldehyde), N butyric acid (butanoic acid), d or 1 carvone (carvol), cinnamaldehyde (cinnamic aldehyde), citral (2,6 dimethyloctadien 2,6 al 8, gera nial, neral), decanal (N decylaldehyde, capraldehyde, capric aldehyde, caprinaldehyde, aldehyde C 10), ethyl acetate, ethyl butyrate, 3 methyl 3 phenyl glycidic acid ethyl ester (ethyl methyl phenyl glycidate, strawberry aldehyde, C16 aldehyde), ethyl vanillin, geraniol (3,7 dimethyl 2,6 and 3,6 octadien 1 ol), geranyl acetate (geraniol acetate), limonene (d, 1, and dl), linalool (linalol, 3,7 dimethyl 1,6 octadien 3 ol), linalyl acetate (bergamol), methyl anthranilate (methyl 2 aminobenzoate), piperonal (3,4 methylenedioxy benzaldehyde, heliotropin), vanillin, alfalfa (*Medicago sativa* L.), allspice (*Pimenta officinalis),* ambrette seed (*Hibiscus abelmoschus*), angelic (*Angelica archangelica*), Angostura (*Galipea officinalis),* anise (*Pimpinella anisum*), star anise (*Illicium verum*), balm (*Melissa officinalis*), basil (*Ocimum basilicum*), bay (*Laurus nobilis*), calendula (*Calendula officinalis*), (*Anthemis nobilis*), capsicum (*Capsicum frutescens*), caraway (*Carum carvi*), cardamom (*Elettaria cardamomum*), cassia (*Cinnamomum cassia*), cayenne pepper (*Capsicum frutescens*), Celery seed (*Apium graveolens*), chervil (*Anthriscus cerefolium*), chives (*Allium schoenoprasum*), coriander (*Coriandrum sativum),* cumin (*Cuminum cyminum*), elder flowers (*Sambucus canadensis*), fennel (*Foeniculum vulgare),* fenugreek (*Trigonella foenum graecum*), ginger (*Zingiber officinale*), horehound (*Marrubium vulgare*), horseradish (*Armoracia lapathifolia*), hyssop (*Hyssopus officinalis*), lavender (*Lavandula officinalis*), mace (*Myristica fragrans*), marjoram (*Majorana hortensis*), mustard (*Brassica nigra*, *Brassica juncea, Brassica hirta*), nutmeg (*Myristica fragrans*), paprika (*Capsicum annuum*), black pepper (*Piper nigrum*), peppermint (*Mentha piperita*), poppy seed (*Papayer somniferum*), rosemary (*Rosmarinus officinalis*), saffron (*Crocus sativus*), sage (*Salvia officinalis*), savory (*Satureia hortensis, Satureia montana*), sesame (*Sesamum indicum*), spearmint (*Mentha spicata*), tarragon (*Artemisia dracunculus*), thyme (*Thymus vulgaris, Thymus serpyllum*), turmeric (*Curcuma longa*), vanilla (*Vanilla planifolia*), zedoary (*Curcuma zedoaria*), sucrose, glucose, saccharin, sorbitol, mannitol, aspartame. Other suitable flavoring are disclosed in such references as Remington's Pharmaceutical Sciences, 18th Edition, Mack Publishing, p. 1288-1300 (1990), and Furia and Pellanca, Fenaroli's Handbook of Flavor Ingredients, The Chemical Rubber Company, Cleveland, Ohio, (1971), known to those skilled in the art.

In other embodiments, the compositions comprise at least one synthetic or natural food coloring (e.g., annatto extract, astaxanthin, beet powder, ultramarine blue, canthaxanthin, caramel, carotenal, beta carotene, carmine, toasted cottonseed flour, ferrous gluconate, ferrous lactate, grape color extract, grape skin extract, iron oxide, fruit juice, vegetable juice, dried, tagetes meal, carrot oil, corn endosperm oil, paprika, paprika oleoresin, riboflavin, saffron, tumeric, tumeric and oleoresin).

In still further embodiments, the compositions comprise at least one phytonutrient (e.g., soy isoflavonoids, oligomeric proanthcyanidins, indol 3 carbinol, sulforaphone, fibrous ligands, plant phytosterols, ferulic acid, anthocyanocides, triterpenes, omega 3/6 fatty acids, conjugated fatty acids such as conjugated linoleic acid and conjugated linolenic acid, polyacetylene, quinones, terpenes, cathechins, gallates, and quercitin). Sources of plant phytonutrients include, but are not limited to, soy lecithin, soy isoflavones, brown rice germ, royal jelly, bee propolis, acerola berry juice powder, Japanese green tea, grape seed extract, grape skin extract, carrot juice, bilberry, flaxseed meal, bee pollen, ginkgo biloba, primrose (evening primrose oil), red clover, burdock root, dandelion, parsley, rose hips, milk thistle, ginger, Siberian ginseng, rosemary, curcumin, garlic, lycopene, grapefruit seed extract, spinach, and broccoli.

In still other embodiments, the compositions comprise at least one vitamin (e.g., vitamin A, thiamin (B1), riboflavin (B2), pyridoxine (B6), cyanocobalamin (B12), biotin, retinoic acid (vitamin D), vitamin E, folic acid and other folates, vitamin K, niacin, and pantothenic acid). In some embodiments, the particles comprise at least one mineral (e.g., sodium, potassium, magnesium, calcium, phosphorus, chlorine, iron, zinc, manganese, flourine, copper, molybdenum, chromium, and iodine). In some particularly preferred embodiments, a dosage of a plurality of particles includes vitamins or minerals in the range of the recommended daily allowance (RDA) as specified by the United States Department of Agriculture. In still other embodiments, the particles comprise an amino acid supplement formula in which at least one amino acid is included (e.g., 1-carnitine or tryptophan).

In one embodiment the sample of animal feed comprises an additive and in one embodiment the additive comprises an enzyme.

In some embodiments, the feed compositions contain supplemental enzymes. Enzymes optimize the nutrient availability of feeding stuffs of plant origin. Monogastric animals e.g. pigs or poultry do not have their own enzymes to utilize specific substances such as non-starch polysaccharides (NSP) and phytates. Therefore, part of the feed is normally not digested. Such undigested constituents pass through the intestinal tract which means the animal losses out on some nutritional value of the feed, plus there is an additional burden on the environment, especially in densely farmed areas. Adding feed enzymes to the diet overcomes this problem and increases the efficiency of nutrient utilization. Exemplary of such enzymes are proteases, fungal proteases, cellulases, xylanases, phytase, acid phosphatases, beta-glucanase, pectinase, and alpha amylase. Enzymes may be provided in purified form, partially purified form, or crude form. Enzyme sources may be nature (e.g., fungal) or synthetic or produced *in vitro* (e.g., recombinant). In some embodiments, a protease (e.g., pepsin) is added. In some embodiments, commercially available enzyme or enzyme mixtures are added (e.g., Allzyme SSF, available from Alltech, Nicholasville, KY).

In some embodiments, antioxidants can also be added to the foodstuff, such as an animal feed composition. Oxidation can be prevented by the introduction of naturally-occurring antioxidants, such as beta-carotene, , vitamin C, and or of synthetic antioxidants such as butylated hydroxytoluene, butylated hydroxyanisole, tertiary-butylhydroquinone, propyl gallate or ethoxyquin to the foodstuff. Compounds which act synergistically with antioxidants can also be added such as ascorbic acid, citric acid, and phosphoric acid. The amount of antioxidants incorporated in this manner depends on requirements such as product formulation, shipping conditions, packaging methods, and desired shelf-life.

Compositions of the invention can be fed to any animal. Exemplary animals include, but are not limited to, avian, bovine, porcine, equine, ovine, and caprine, piscines, shellfish, camelids, feline, canine, and rodent species. Thus, in some embodiments, a foodstuff comprising a dietary supplement composition of the invention is fed to any monogastric animal (i.e., an animal having a stomach with a single compartment) including but not limited to agricultural animals, such as porcine species (e.g., barrows (i.e., castrated male pigs), gilts (i.e., female pigs prior to first mating) and any other type of swine), chickens (e.g., any type, kind, or species including but not limited to broilers and breeders), turkeys (poults (i.e., first several weeks post-hatching) and older animals), ducks, pheasants, geese, quail, cattle, sheep, goats, laboratory rodents (rats, mice, hamsters and gerbils), fur-bearing animals such as mink and fox, and zoo animals such as monkeys and apes, any other avian species, marine or fresh water aquatic species, animals held in captivity (e.g., zoo animals), or domestic animals (e.g., canine and feline).

### III. Systems

Systems for analysis of animal feed comprise components useful, necessary, or sufficient for performing in vitro digestion, obtaining spectral data, and analyzing the spectral data to determine impact of feed additives on digestion of the animal feed.

For example, systems comprise reagents and vessels for performing in vitro digestion (e.g., enzymes, acid, temperature control components, reaction vessels, etc.). In some cases, systems comprise NIR instruments and consumables. In some cases, systems comprise computer systems and computer software for analyzing spectral data.

A variety of computer-related implementations are possible such as computer programming for analyzing and comparing a pattern of NIR peaks to, for example, a library of peaks, known to represent a particular analyte (e.g., using the methods described herein).

The methods and systems described herein can be implemented in numerous ways. The methods involve use of a communications infrastructure, for example the internet. Several examples of the invention are discussed below. It is also to be understood that the present invention may be implemented in various forms of hardware, software, firmware, processors, distributed servers (e.g., as used in cloud computing) or a combination thereof. The methods and systems described herein can be implemented as a combination of hardware and software. The software can be implemented as an application program tangibly embodied on a program storage device, or different portions of the software implemented in the user's computing environment (e.g., as an applet) and on the reviewer's computing environment, where the reviewer may be located at a remote site (e.g., at a service provider's facility).

For example, during or after data input by the user, portions of the data processing can be performed in the user-side computing environment. For example, the user-side computing environment can be programmed to provide for defined test codes to denote platform, carrier/diagnostic test, or both; processing of data using defined flags, and/or generation of flag configurations, where the responses are transmitted as processed or partially processed responses to the reviewer's computing environment in the form of test code and flag configurations for subsequent execution of one or more algorithms to provide a results and/or generate a report in the reviewer's computing environment.

The application program for executing the algorithms described herein may be uploaded to, and executed by, a machine comprising any suitable architecture. In general, the machine involves a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the application program (or a combination thereof) which is executed via the operating system. In addition, various other peripheral devices may be connected to the computer platform such as an additional data storage device and a printing device.

As a computer system, the system generally includes a processor unit. The processor unit operates to receive information, which generally includes test data (e.g., NIR spectra), and a database of known data (e.g., experimentally determined peak information from a plurality of samples). This information received can be stored at least temporarily in a database, and data analyzed.

Part or all of the input and output data can also be sent electronically; certain output data (e.g., reports) can be sent electronically or telephonically (e.g., by facsimile, e.g., using devices such as fax back). Exemplary output receiving devices can include a display element, a printer, a facsimile device and the like. Electronic forms of transmission and/or display can include email, interactive television, and the like. In some embodiments, all or a portion of the input data and/or all or a portion of the output data (e.g., peak identification and quantitation) are maintained on a server for access, e.g., confidential access. The results may be accessed or sent to professionals as desired.

A system for use in the methods described herein generally includes at least one computer processor (e.g., where the method is carried out in its entirety at a single site) or at least two networked computer processors (e.g., where spectral data is to be input by a user) and transmitted to a remote site to a second computer processor for analysis (e.g., identification and characterization of peaks), where the first and second computer processors are connected by a network, e.g., via an intranet or internet). The system can also include a user component(s) for input; and a reviewer component(s) for review of data, and generation of reports. Additional components of the system can include a server component(s); and a database(s) for storing data (e.g., as in a database of report elements, or a relational database (RDB) which can include data input by the user and data output). The computer processors can be processors that are typically found in personal desktop computers (e.g., IBM, Dell, Macintosh), portable computers, mainframes, minicomputers, portable electronic devices (e.g., tablets or smart phones) or other computing devices.

The input components can be complete, stand-alone personal computers offering a full range of power and features to run applications. The user component usually operates under any desired operating system and includes a communication element (e.g., a modem or other hardware for connecting to a network), one or more input devices (e.g., a keyboard, mouse, keypad, or other device used to transfer information or commands), a storage element (e.g., a hard drive or other computer-readable, computer-writable storage medium), and a display element (e.g., a monitor, television, LCD, LED, or other display device that conveys information to the user). The user enters input commands into the computer processor through an input device. Generally, the user interface is a graphical user interface (GUI) written for web browser applications.

The server component(s) can be a personal computer, a minicomputer, or a mainframe, or distributed across multiple servers (e.g., as in cloud computing applications) and offers data management, information sharing between clients, network administration and security. The application and any databases used can be on the same or different servers. Other computing arrangements for the user and server(s), including processing on a single machine such as a mainframe, a collection of machines, or other suitable configuration are contemplated. In general, the user and server machines work together to accomplish the processing of the present invention.

Where used, the database(s) is usually connected to the database server component and can be any device which will hold data. For example, the database can be any magnetic or optical storing device for a computer (e.g., CDROM, internal hard drive, tape drive). The database can be located remote to the server component (with access via a network, modem, etc.) or locally to the server component.

Where used in the system and methods, the database can be a relational database that is organized and accessed according to relationships between data items. The relational database is generally composed of a plurality of tables (entities). The rows of a table represent records (collections of information about separate items) and the columns represent fields (particular attributes of a record). In its simplest conception, the relational database is a collection of data entries that "relate" to each other through at least one common field.

Additional workstations equipped with computers and printers may be used at point of service to enter data and, in some embodiments, generate appropriate reports, if desired. The computer(s) can have a shortcut (e.g., on the desktop) to launch the application to facilitate initiation of data entry, transmission, analysis, report receipt, etc. as desired.

### EXPERIMENTAL

The following examples are provided in order to demonstrate and further illustrate certain preferred embodiments and aspects of the present invention and are not to be construed as limiting the scope thereof.

### Example 1

In Vitro digestion

### Swine In vitro Model for Phosphate and Sugar Release

**Reference:** Boisen S., A multienzyme assay for pigs, Chapter 10, A Model for Feed Evaluation Based on Invitro Digestible Dry Matter and Protein, Invitro Digestion for Pig and Poultry, 1990, M.F. Fuller

### Reagents:

A. 0.2 M HCl
B. 4 M HCl
C. 2M HCl
D. 0.6 M NaOH: dissolve 24g sodium hydroxide (Fisher S318) in 900ml De-ionized water then bring to final volume of 1L.
E. Pepsin from Sigma (P7012): store at -20°C
F. Pancreatin from Sigma (P3292): store at -20°C
G. 15% trichloroacetic acid (TCA): dilute 15g trichloroacetic acid (Sigma T6399) with de-ionized water to a volume of 100ml
H. 0.1 M Acetate Buffer, pH 6.0
a. Dissolve 8.203 g of sodium acetate (Fisher S210) in 900ml of de-ionized water
b. Adjust to pH 6.0 with 1M HCL and bring to 1L volume with de-ionized water

I. 0.2 M Acetate Buffer, pH 6.8
a. Dissolve 16.406 g of sodium acetate (Fisher S210) in 900ml of de-ionized water
b. Adjust to pH 6.8 and bring to 1L volume with de-ionized water

J. Color Reagent (make fresh)
a. 3 volumes of 1 M Sulfuric Acid
   i. In a volumetric flask, add 5.52ml concentrated (18.1M) sulfuric acid (S6014) to 90ml de-ionized water, then bring to a final volume of 100ml
      1. 40 mL of 5N Sulfuric Acid added to 60 mL of de-ionized water
b. 1 volume of 2.5% (w/v) Ammonium Molybdate
   i. Dissolve 2.65g of ammonium molybdate tetrahydrate (Sigma A7302) in 80ml de-ionized water, then bring to a final volume of 100ml
c. 1 volume of 10% (w/v) Ascorbic Acid
   i. Dissolve 10g ascorbic acid (Fisher BP351) into 80ml de-ionized water, then bring to a final volume of 100ml

K. DNS solution: store in dark bottle-good for 6 months
a. Dissolve 10 g dintrosalysilic acid (Sigma D0550) in 400 ml
b. Dissolve 16 g NaOH (Fisher S318) in 150 ml de-ionized water
c. Add NaOH solution slowly to dintrosalicylic acid solution while stirring
d. Place in 50°C water bath till all solids dissolve
e. Add 300 g potassium sodium tartrate tetrahydrate (Sigma 217255) while stirring
f. Bring to 1L volume with de-ionized water

L. Dextrose standards: dilute 1g dextrose (Fisher D16) with de-ionized water to make a volume of 100ml. From this stock solution create dextrose dilutions of the following:

| **Dilution of 1g/100ml stock solution** | **Dextrose Concentration (mg/ml)** |
|---|---|
| 0 | 0 |
| 1:50 | 0.2 |
| 1:25 | 0.4 |
| 1:16.67 | 0.6 |
| 1:12.5 | 0.8 |
| 1:10 | 1.0 |

M. 9 mM Potassium Phosphate (KH₂PO₄) solution - for standard curve
a. Dissolve 1.22g of KH₂PO₄ (Fisher Sp361) into 800ml de-ionized water, then bring to a final volume of 1L

N. Phosphate standards: make the following phosphate dilutions with the 9mM phosphate stock solution:

| **Dilution of 9.0mM Phosphate Stock** | **Phosphate Concentration (µM)** |
|---|---|
| 0 | 0 |
| 1:1600 | 5.625 |
| 1:800 | 11.25 |
| 1:400 | 22.5 |
| 1:200 | 45 |
| 1:100 | 90 |

### Procedure

### SSF Extraction

1. 1 g SSF-wheat bran added to 100 mL of de-ionized water in a 125 mL bottle with cap
2. Shake at 250 for 1 hr
3. Dilute
   a. 1:500 (1 mL SSF/ 4 mL 0.1M sodium acetate buffer) then 1:5000 (1 mL 1:500 mix/ 9 mL buffer)

### Step 1 - Stomach

1. Grind feed and pass ground feed through a 1x1mm sieve
2. Add 2 g sample in 250 ml flask
   a. 50 minutes into extracting SSF, add 50 ml 0.1M sodium acetate solution and slowly add 20 ml 0.2M HCl while stirring
   b. When no enzyme in feed add 1 ml enzyme + 49 ml sodium acetate
3. Adjust to pH 3 with 4M HCl(∼10 drops), then pH 2 with 2M HCl (∼10 drops)
4. Add 2 ml pepsin solution (10mg pepsin/ml de-ionized water, make fresh) and 1.0 ml chloramphenicol solution(5mg chloramphenicol (Sigma C0378)/ 1 ml alcohol, make fresh)
   a. Example: Pepsin = 130 mg/13mL of D.I. water (6 flasks + 1 mL extra)
   b. Example: Chloramphenicol = 32.5 mg/ 6.5 mL of alcohol (6 flasks + 0.5 mL extra)
5. Cover flasks, stir solution, and place in 39°C agitating water bath (55 RPM) for 6 hrs (use weights to keep flasks from floating)
   a. Every hour stir flasks well to ensure good mixing

### Step 2- Small Intestines

1. Add 20 ml 0.2M sodium acetate buffer and 10 mL 0.6M NaOH (add slowly while stirring)
2. Adjust to pH 6.8 with 0.6M NaOH (20-25 drops)
3. Add 2mL pancreatin solution (50 mg/ml D.I. water + 2 mL extra; mix in beaker with stir bar, make fresh)
4. Stir solution and place in a 39°C agitating (55 RPM) water bath for 18 hours
5. Stir solution, pour into Falcon centrifuge tube, and centrifuge at 14000g for 20 minutes

### Phosphate Release:

Reference: Kim T.W., Lei X.G. (2005), An Improved Method for a Rapid Determination of Phytase in Animal Feed, J. Animal science, 83:1062-1067
1. After incubation, pipette 1ml of 15% TCA stop solution into 1ml supernatant just before centrifuge is complete (use duplicates), then vortex tubes
2. Centrifuge the tubes at 2,000x g for 10 minutes.
   a. Color Reagent can be prepared during this time
3. In clean test tubes, mix 0.2 ml of the supernatant with 1.8ml of nanopure water (18MΩ·cm). Also add 0.2ml of each phosphate standard to 1.8ml of nanopure water and vortex. While adding supernatant, burn one sample of each before adding to tubes.
4. Add 2.0 ml of fresh Color Reagent to all tubes and vortex well.
5. Incubate all samples in a water bath a 50°C for 15 minutes.
6. Allow all samples to come to room temperature. Place thermometer in room temperature water bath (20-25°C).
7. Read the absorbance at 820 nm.
   a. Compare absorbance averages to standards and calculate mg phosphate/kg feed
   b. remember to take into account the dilution when calculating results

### Reducing Sugar Release:

Reference: Miller (1959), Use of Dintrosalicylic Acid Reagent for Determination of Reducing Sugar, Anal. Chem. 31, 426-428
1. Dilute 1ml supernatant with de-ionized water if necessary
2. Place 1ml of diluted supernatant in 1ml water in a glass test tube (use duplicates)
3. Pipette 1ml of each dextrose standard solution into 1ml water in glass test tube
4. Add 3ml DNS solution to each test tube and immediately vortex and place in boiling water bath for 5 minutes
5. Place boiled test tubes in ice water bath for about 2-3 minutes then let the tubes reach room temperature. Place thermometer in room temperature water bath (20-25°C).
6. Read the absorbance at 540nm
   a. Compare absorbance averages to standards and calculate g reducing sugars/kg feed
   b. remember to take into account the dilution when calculating results

### Dilution of Enzyme:

1. Determine dilution factor
2. Dilute 1:100 (1g/100ml) in DI water
3. Mix for 60min (RT, 250RPM) in Peptone bottles
4. Continue dilutions in 15ml tubes
5. Refer to step 1.3

The in vitro procedure leaves a final mass consisting of digested feed and the liquid components that have been added. This mixture was filtered using grade P8 filter paper and a Buchner funnel to obtain the solid components. The liquid portion was analyzed for phosphorous content using an ICP system while the solid portion was freeze dried. This freeze dried mass gives the final dry matter portion. The dry matter is scanned using NIR for calibration. A portion of the dry matter is also tested for protein content using a nitrogen combustion analyzer. Another portion is tested on a bomb calorimeter for gross energy. Table 1 shows an exemplary dataset for a feed that has undergone in vitro digestion and analysis.

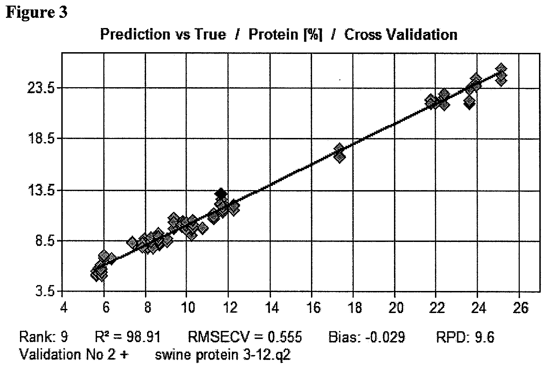

A model was generated using NIR and the data obtained from the other analytical tools as reference data. NIR spectra consist of peaks that relate to certain bonds within a substance's structure (e.g., protein has its own peaks). These peaks are then related back to the reference data acquired so that after multiple spectra and data points have been obtained a prediction model is created for a particular component of the feed (e.g., enzyme). Figure 2 shows an exemplary NIR spectrum. Figure 3 shows the accuracy for a NIR prediction of residual protein concentration.

**Table 1**

| **Name** | **Diet Used** | **% Protein** | **avg** | **Phosphorus Release** | **avg** | **Gross Energy** | **avg** | **P Remain** | **avg** |
|---|---|---|---|---|---|---|---|---|---|
| | | | | | | | | | |
| Flask 1 | Soybean Meal | 5.8255625 | 6.077813 | 964.9294136 | 972.3235 | 4283.47 | 4239.233 | 1046.38 | 1038.987 |
| Flask 2 | Soybean Meal | 6.405 | | 987.1116922 | | 4191.84 | | 1024.2 | |
| Flask 3 | Soybean Meal | 6.002875 | | 964.9294136 | | 4242.39 | | 1046.38 | |
| Flask +enzyme 4 | Soybean Meal | 5.6355 | 5.773125 | 1353.119289 | 1314.916 | 4263.09 | 4267.5 | 658.19 | 696.3933 |
| Flask +enzyme 5 | Soybean Meal | 5.7609375 | | 1312.451778 | | 4272.13 | | 698.86 | |
| Flask +enzyme 6 | Soybean Meal | 5.9229375 | | 1279.17836 | | 4267.28 | | 732.13 | |

Various modifications and variations of the described compositions and methods of the invention will be apparent to those skilled in the art. Although the invention has been described in connection with specific preferred embodiments, it should be understood that the invention as claimed should not be unduly limited to such specific embodiments. Indeed, various modifications of the described modes for carrying out the invention that are obvious to those skilled in the relevant fields are intended to be within the scope of the appended claims.

## Claims

1. A method of analyzing animal feed comprising the step of:
a) digesting a sample of animal feed in vitro using at least one digestive enzyme to generate digested animal feed comprising at least one residual component;
**characterized by**:
b) scanning the digested animal feed using NIR spectroscopy to generate spectral data; and
c) comparing the spectral data to a computer model to generate a predicted concentration of the at least one residual component of the digested animal feed.

2. The method of claim 1, wherein at least one digestive enzyme is pepsin or pancreatin or both.

3. The method of claim 1 or claim 2, wherein the digesting the sample of animal feed further comprises separating the digested sample into a dry matter portion and a liquid portion and wherein scanning the digested animal feed comprises scanning the dry matter portion.

4. The method of claim 3, wherein the dry matter portion is dried before scanning the dry matter portion.

5. The method of any one of claims 1-4, wherein the at least one residual component is selected from the group consisting of protein, phosphorous, gross energy, and carbohydrates.

6. The method of any one of claims 1-5, wherein the sample of animal feed comprises an additive.

7. The method of claim 6, wherein the additive comprises an enzyme.

8. The method of any one of claims 1-7, wherein the method further comprises determining the effect of the additive on digestibility of the animal feed by comparing the predicted concentration of the at least one residual component in the sample of digested animal feed comprising the additive to a predicted concentration of the at least one residual component in a sample of the digested animal feed without the additive.

9. The method of any one of claims 1-8, wherein the spectral data is compared using a computer implemented method comprising receiving spectral data from the digested sample and comparing the spectral data to the computer model to obtain the predicted concentration of the at least one residual component.

## Patentansprüche

1. Verfahren zum Analysieren von Tierfutter umfassend den Schritt:
a) Verdauen einer Probe von Tierfutter in vitro unter Verwendung von mindestens einem Verdauungsenzym, um verdautes Tierfutter, umfassend mindestens eine restliche Komponente, zu erzeugen;
**gekennzeichnet durch**:
b) Scannen des verdauten Tierfutters unter Verwendung von NIR-Spektroskopie, um Spektraldaten zu erzeugen; und
c) Vergleichen der Spektraldaten mit einem Computermodell, um eine vorhergesagte Konzentration der mindestens einen restlichen Komponente des verdauten Tierfutters zu erzeugen.

2. Verfahren nach Anspruch 1, wobei mindestens ein Verdauungsenzym Pepsin oder Pankreatin oder beides ist.

3. Verfahren nach Anspruch 1 oder Anspruch 2, wobei das Verdauen der Probe von Tierfutter ferner das Trennen der verdauten Probe in einen Trockensubstanzanteil und einen Flüssiganteil umfasst und wobei das Scannen des verdauten Tierfutters das Scannen des Trockensubstanzanteils umfasst.

4. Verfahren nach Anspruch 3, wobei der Trockensubstanzanteil vor dem Scannen des Trockensubstanzanteils getrocknet wird.

5. Verfahren nach einem der Ansprüche 1 bis 4, wobei die mindestens eine restliche Komponente ausgewählt ist aus der Gruppe bestehend aus Protein, Phosphor, Bruttoenergie und Kohlenhydraten.

6. Verfahren nach einem der Ansprüche 1 bis 5, wobei die Probe des Tierfutters einen Zusatzstoff umfasst.

7. Verfahren nach Anspruch 6, wobei der Zusatzstoff ein Enzym umfasst.

8. Verfahren nach einem der Ansprüche 1 bis 7, wobei das Verfahren ferner das Bestimmen der Wirkung des Zusatzstoffs auf die Verdaulichkeit des Tierfutters umfasst, durch Vergleichen der vorhergesagten Konzentration der mindestens einen restlichen Komponente in der Probe des verdauten Tierfutters, umfassend den Zusatzstoff, mit einer vorhergesagten Konzentration der mindestens einen restlichen Komponente in einer Probe des verdauten Tierfutters ohne den Zusatzstoff.

9. Verfahren nach einem der Ansprüche 1 bis 8, wobei die Spektraldaten unter Verwendung eines computerimplementierten Verfahrens verglichen werden, umfassend das Empfangen von Spektraldaten von der verdauten Probe und das Vergleichen der Spektraldaten mit dem Computermodell, um die vorhergesagte Konzentration der mindestens einen restlichen Komponente zu erhalten.

## Revendications

1. Procédé d'analyse d'aliment pour animaux comprenant l'étape de :
a) digestion d'un échantillon d'aliment pour animaux *in vitro* en utilisant au moins une enzyme digestive pour générer l'aliment pour animaux digéré comprenant au moins un constituant résiduel ;
**caractérisé par**
b) le balayage de l'aliment pour animaux digéré en utilisant la spectroscopie NIR pour générer des données spectrales ; et
c) la comparaison des données spectrales à un modèle informatique pour générer une concentration prédite dudit constituant résiduel de l'aliment pour animaux digéré.

2. Procédé selon la revendication 1, dans lequel au moins une enzyme digestive est la pepsine ou la pancréatine ou les deux.

3. Procédé selon la revendication 1 ou la revendication 2, la digestion de l'échantillon d'aliment pour animaux comprenant en outre la séparation de l'échantillon digéré en une partie matière sèche et une partie liquide et où le balayage de l'aliment pour animaux digéré comprend le balayage de la partie matière sèche.

4. Procédé selon la revendication 3, la partie matière sèche étant séchée avant le balayage de la partie matière sèche.

5. Procédé selon l'une quelconque des revendications 1 à 4, dans lequel ledit constituant résiduel est sélectionné dans le groupe constitué des protéines, du phosphore, de l'énergie brute, et des hydrates de carbone.

6. Procédé selon l'une quelconque des revendications 1 à 5, dans lequel l'échantillon d'aliment pour animaux comprend un additif.

7. Procédé selon la revendication 6, dans lequel l'additif comprend une enzyme.

8. Procédé selon l'une quelconque des revendications 1 à 7, dans lequel le procédé comprend en outre la détermination de l'effet de l'additif sur la digestibilité de l'aliment pour animaux en comparant la concentration prédite dudit constituant résiduel dans l'échantillon d'aliment pour animaux digéré comprenant l'additif à une concentration prédite dudit constituant résiduel dans un échantillon de l'aliment pour animaux digéré sans l'additif.

9. Procédé selon l'une quelconque des revendications 1 à 8, dans lequel les données spectrales sont comparées en utilisant un procédé mis en oeuvre par ordinateur comprenant la réception de données spectrales de l'échantillon digéré et la comparaison des données spectrales au modèle informatique pour obtenir la concentration prédite dudit constituant résiduel.
